Europäisches Patentamt

European Patent Office

Office européen des brevets

(11) Publication number: 0 369 533

A2

## (12) EUROPEAN PATENT APPLICATION

(21) Application number: 89202846.5

(22) Date of filing: 09.11.89

(51) Int. Cl.⁵: **C07C 31/28, C07C 43/13, C07C 215/08, H01L 39/24**

(30) Priority: **14.11.88 US 270551**

(43) Date of publication of application:
**23.05.90 Bulletin 90/21**

(84) Designated Contracting States:
**DE FR GB**

(71) Applicant: **AKZO N.V.**
**Velperweg 76**
**NL-6824 BM Arnhem(NL)**

(72) Inventor: **Whitwell, George Edward**
**R1, Box 48 Maybrook Road**
**Campbell Hall New York 10916(US)**
Inventor: **Deatcher, John Hutcheson**
**160 Walnut Road**
**Lake Peekshill New York 10537(US)**
Inventor: **Burkhardt, Eric William**
**46 Juniper Circle**
**Brewster New York 10509(US)**

(74) Representative: **Sieders, René et al**
**AKZO N.V. Patent Department (Dept. CO)**
**P.O. Box 9300**
**NL-6800 SB Arnhem(NL)**

(54) Solubilized metal alkoxides in alkoxyalkanol solvent.

(57) Metal alkoxides, including those not containing ligands derived from an alkoxy alcohol, can be solubilized in an alkoxyalkanol solvent. The resulting solution is useful in the fabrication of metal oxide films on a substrate.

# SOLUBILIZED METAL ALKOXIDES IN ALKOXYALKANOL SOLVENT

## BACKGROUND OF THE INVENTION

The present invention relates to the use of alkoxyalkanols as solvents to form metal alkoxides. The solvent systems thus formed can be used to form metal oxide thin films for electronic applications.

It is known to form thin film coatings of metal oxides by spin coating techniques. For example, "Active and Passive Thin Film Devices", T. J. Coutts, ed. (Academic Press, London, 1978) indicates, on page 51, that oxide films can be formed using organic solution techniques and spin-on procedures. More recently, K. Y. Tsao, J. Electrochemical Soc., Vol. 132, No. 3 (March 1985), Paper No. 226, mentioned the use of a spin-on technique in forming a borophosphosilicate glass as a dielectric layer.

Certain metal alkoxides (e.g., transition metal alkoxides), if dissolved in certain solvents (e.g., xylene-based solvents), produce films which are deficient in various respects (e.g., they are flaky, white, and/or non-adherent). Therefore, a need exists for means to increase the solubilization of such difficultly soluble metal alkoxide species for film formation. One recent example of a teaching in regard to how the solubility of metal alkoxides might be increased is described in U.S. Patent No. 4,634,786 to C. W. Kamienski wherein magnesium dialkoxides were made more soluble by using an alkoxide ligand derived from certain 2-alkoxyalkanols. These magnesium alkoxyalkanol species were formed by mixing only slightly more than the stoichiometric amount of alkoxyalkanol needed for reaction with the magnesium dialkoxide starting material with the ultimate solvent medium being a halogenated hydrocarbon solvent. The halogenated hydrocarbon was therefore present in substantially greater stoichiometric amount than the magnesium dialkoxide starting material.

More recently, European Patent Publication Nos. 244,916 and 244,917 listed certain alkoxyalkanols for use as solvents with certain organic solvent soluble zinc alkoxy alkoxides and certain organic solvent soluble polyvalent metal (e.g., magnesium, calcium, strontium, barium, scandium, yttrium, and lanthanum) alkoxy alkoxides. In all cases where an alkoxy alcohol was contemplated for use by these two patent publications, the starting metal alkoxide had ligands also derived from an alkoxy alcohol. These patent publications do not ascribe any superiority for an alkoxy alcohol solvent over the other solvents listed.

## SUMMARY OF THE INVENTION

The instant invention relates to the use of an alkoxyalkanol as the solvent medium for metal alkoxides which have ligands derived from an alcohol other than an alkoxy alcohol unlike the metal alkoxy alkoxides described in European Patent Publication Nos. 244,916 and 244,917. In the present invention the alkoxy alcohol solvent is present in substantially higher stoichiometric amount than the metal alkoxide desired to be solubilized as compared to the amount mentioned for use in U.S. Patent No. 4,634,786.

## DETAILED DESCRIPTION OF THE INVENTION

The metal alkoxides which form the solute in the compositions of the present invention can be selected from Groups IIA-VA and Groups IB-VB of the Periodic Table of Elements. Representative compounds include such alkaline earth metals as barium, magnesium and strontium, such Group IIIA elements as aluminum, such Group IVA elements as silicon, such Group IIIB elements as yttrium, and such Group IVB elements as zirconium and titanium. The alkoxide ligands are not derived from alkoxy alcohols and can be chosen from either $C_2$ to $C_7$ alkyl, such as ethoxide, isopropoxide, n-propoxide, or n- or sec-butoxide, aryl or substituted aryl, alkylamino, or dialkylamino. Generally speaking, the alkoxides with alkyl or aryl ligands will be insoluble in most solvents or only sparingly soluble while the alkoxides with aminoalkanol-derived ligands (especially dialkylamino ligands) may be more soluble, perhaps substantially soluble. For example, certain bis(aminoalkoxy) and bis(alkylaminoalkoxy)copper(II) compounds of the formulae $Cu(ORNH_2)_2$ and $Cu(ORNHR')_2$, where R is $C_2$-$C_4$ alkylene and R' is $C_1$-$C_4$ alkyl can be used with the present invention. In addition, the more desirable aminoalcoholate-derived compounds of the formula $M(ORNR'_2)_x$, where M is a superconductor metal precursor (Cu, Y, Ba, Ca, Bi, La, Sr, the rare earths, and the like), R is $C_2$-$C_3$ alkylene, R' is $C_1$-$C_8$ alkyl and x is the valence of the metal can also be used in regard to the instant invention. Generally speaking, the total number of carbon atoms in each of the alkoxide ligands can range from about 1 to about 6. It may be either straight or branched.

The alkoxyalkanol solvent is present in substantial molar excess to the metal alkoxide solute, e.g., below the limit of saturation of the alkoxide in the solvent. Preferably, solutions containing 5 to 10 wt % metal oxide equivalent are contemplated. Representative alkoxyalkanols have a total of from about 3 to about 8 carbon atoms. The alkoxy group can be a $C_1$-$C_4$ alkoxy substituent. Representative alkoxyalkanols are methoxypropanol, methoxybutanol, methoxy ethanol, and butoxyethanol.

The solutions of this invention can contain the metal alkoxides either singly or in admixture. In either case, the solutions can be used to coat a substrate by means of the spin coating a curing process described below and then can be heated (fired) to form a metal oxide film on the substrate. The solution may be prehydrolyzed or hydrolysis may occur during film drying via atmospheric moisture. It is possible to select the types and amounts of the metal moieties so that complex (polymetallic) metal oxides including mixed metal oxide high temperature superconductor films can be formed.

The following is a description of the spin-coating and curing procedure which can be used.

The spin-coating process involves the application of the metal complex solution onto flat, highly polished wafers. The spin-coating machine employed can either be a Headway Research model number EC-102-NRD or an Integrated Technologies model number P6204. The spin-coating process can be carried out in a laminar flow hood or in an inert atmosphere box. The films can be generated by flooding the wafer with the formulation and removing the excess formulation by rapid acceleration to the desired angular velocity (1,000-10,000 rpm). Spinning times can generally be from 20-60 seconds. Variation of spin speed results in variation of the resulting film thickness.

The wet films can then be cured either by placement onto a hotplate at 420°C for 5 minutes or by passing through an infrared heating furnace on a conveyor belt with a maximum temperature of 350°C for 20 minutes. Multiple coating operations can also be carried out in which the wet films are cured using an infrared furnace at a temperature of 270°C for 5 minutes between coatings. Generally 3 to 5 coatings can be applied in this manner before a higher temperature cure is required in order to preserve good film quality.

The present invention is further illustrated by the Examples which follow. In some of the examples the characteristics of systems derived from metal alkoxides containing ligands derived from alkoxy alcohols is provided for comparative purposes.

## EXAMPLE 1

Metal oxide thin films were prepared by spinning solutions of metal alkoxides in various organic solvents. The solutions were protected from air until dispensed on the substrate. The spinning was done onto silicon wafers in air, e.g. at about 2500 rpm, and was followed by a 420°C hot plate cure for 5 minutes. Good quality films were uncracked and highly reflective. The results:

| Alkoxide/Solvent | Type of Film |
|---|---|
| Yttrium isopropoxide (5%) in xylenes | Flaky, white, nonadherent |
| Yttrium isopropoxide (5%) in 1-methoxy-2-propanol | Good films |
| Yttrium methoxypropoxide (7%) in methoxypropanol | Cracked films |
| Barium methoxypropoxide (6%) in methoxypropanol | Good films |
| Yttrium methoxypropoxide/barium methoxypropoxide 1:2 (6.5%) in methoxypropanol | Good films |
| Yttrium methoxypropoxide/barium methoxypropoxide/copper aminoethoxide 1:2:3 (10%) in methoxypropanol | Good film, solution precipitates in 30 min. |

The above systems are applicable to production of high $T_c$ superconductor films.

## EXAMPLE 2

Metal alkoxides were dissolved in certain organic solvents and spun onto silicon wafers until dry. The spinning was at about 2500 rpm and the films were cured at 420°C for 10 minutes. Good quality films were highly reflective. The following results were obtained:

| Alkoxide/Solvent | Type of Film |
|---|---|
| Zirconium n-butoxide (5%) in methoxypropanol[1] | Reflective films |
| Zirconium n-butoxide (10%) in methoxypropanol[2] | Increasingly reflective with greater water content |
| Titanium n-butoxide (5%) in methoxypropanol[1] | Reflective films |
| Titanium n-butoxide (10%) in methoxypropanol[2] | Increasingly reflective with greater water content |
| Zirconium n-butoxide (5%) in xylenes | Poor films, solution gelled on addition of water |
| Titanium n-butoxide (5%) in xylenes | Good film, gelled with water addition |

(1) with 0-1 eq. of water added.
(2) with 0-2 eq. of water added.

## EXAMPLE 3

Thin films of yttria-zirconia containing from 3-10 weight percent $Y_2O_3$ were produced using commercially available metal alkoxides (yttrium isopropoxide or yttrium methoxypropoxide and zirconium n-propoxide) via standard spin-on techniques. The system can be prepared containing toluene and n-octane or methoxy propanol and n-propanol as co-solvents. The alkoxide solution was further diluted with an alkoxy alcohol solvent (methoxypropanol, methoxybutanol) in order to reduce the total percent metal oxide content (2-9%) and to aid in the production of planar films. Films spun onto 5.1 or 7.6 cm diameter wafers from 1000-5000 rpm for a period of 20-60 seconds are then usually passed through an Intex furnace at temperatures from 270°C (for 5 minutes) to 350°C (for 20 minutes). Single coatings generally range in thickness from 500 Angstroms (for a 2.5 weight percent oxide solution) to 2000 Angstroms (for a 9.2 weight percent oxide solution) when heated to 350°C.

For production of thicker films which have been heated to high temperatures (750°C), multicoat processing must be performed. For example, a solution containing 6.5 weight percent metal oxide is spun at 2000 rpm for 60 seconds, followed by drying in an Intex furnace for 5 minutes at 270°C. The wafer is coated twice more with Intex furnace drying after each coat is applied. After the third coat has been applied and dried, the wafer is heated at 750°C in a tube furnace for 1 hour. The resulting film is highly reflective, adherent (ASTM method D3359) and possessed a thickness of about 2200 Angstroms. The coating procedure may then be repeated as before in order to yield thicker films. Scanning Electron Micrograph analysis of yttria/zirconia films obtained in this manner show planar films with no apparent porosity. X-ray diffraction analysis indicated cubic zirconia as the only crystalline oxide present. Final coating thickness are dependent upon metal oxide content of the solution used, spinning speed, and the number of applications of solution. These films are useful as wear coatings and thermal barriers.

## EXAMPLE 4

A solution containing niobium methoxypropylate was prepared in methoxypropanol containing 5.5 wt % $Nb_2O_5$. The solution was spun at 2000 rpm for 60 seconds followed by heat treatment at 350°C. A good, reflective film of $Nb_2O_5$ was obtained with a thickness of 520 Angstroms.

Other films prepared by the same method include:

| Alkoxide/Solvent | Type of Film |
|---|---|
| Aluminum methoxypropoxide (2-10% oxide) in methoxypropanol | Cloudy films |
| Aluminum sec-butoxide (3-6 wt % oxide) in methoxypropanol | Reflective films |
| Barium methoxypropoxide/titanium isopropoxide (1:1) (3-7 wt % oxide) in methoxypropanol | Clear, reflective films |
| Magnesium methoxypropoxide/aluminum sec-butoxide (1:2) (2-5 wt % oxide) in methoxypropanol | Clear, reflective films |
| Aluminum sec-butoxide/silicon ethoxide (3:2) (2-8 wt % oxide) in xylene | Good reflective films |
| Magnesium methoxypropoxide/aluminum sec-butoxide/silicon ethoxide (2:4:5) (3-5 wt % oxide) in methoxypropanol | Good reflective films |

EXAMPLE 5

Metal alkoxides were dissolved in organic solvents and spun onto silicon wafers until dry. The spinning was at about 2500 rpm and the films were cured at 420° C for 20 minutes. Good films show high reflectivity. The results:

| Alkoxide/Solvent | Type of Film |
|---|---|
| (Strontium isopropoxide (10%) in methoxypropanol[1] | All reflective films |
| Yttrium isopropoxide (2.5-10%) in methoxypropanol[2] | Clear reflective to cloudy films |
| Yttrium isopropoxide (3%) and zirconium n-butoxide (7%) in methoxypropanol[1] | Reflective film |
| Strontium isopropoxide and titanium butoxide (1:1) at 10% in methoxypropanol | Reflective film |
| Barium methoxypropoxide and titanium butoxide (1:1) at 10% in methoxypropanol | Reflective film |
| Yttrium isopropoxide (0.3%) and zirconium butoxide (9.7%) in methoxypropanol | Reflective film |
| Yttrium isopropoxide (0.6%) and zirconium butoxide (9.4%) in methoxypropanol | Reflective film |

(1) with 0.1 eq. of added $H_2O$.
(2) the 10% level gave a cloudy film, the 5% slightly better and the 2.5% a clear reflective film.

EXAMPLE 6

5

A solution of strontium 1-methoxy-2-propoxide (1.33 gm: 5 mmol) and titanium isobutoxide (1.70 gm; 5 mmol) in anhydrous 1-methoxy-2-propanol (23 ml) was prepared in a septum-sealed bottle under argon. Coatings were prepared on 7.6 cm Si wafers under these conditions:

0.5-1.0 ml solution

3000 rpm

30 sec

420° C/ 15 min

R.T./ 15 min

Each wafer was coated 1-4 times by this procedure. The films were subsequently cured at 750° C for 4 hours with a 1 hour warm-up and 15 hour cool down. A portion of each film was etched off with buffered HF and the thickness measured by profilometry (Sloan Dektak 3030 apparatus). The film thicknesses (in Angstroms (A)) were:

1 coat 334 Angstroms

2 coats 633 Angstroms

3 coats 929 Angstroms

4 coats 1270 Angstroms

Strontium titanate films are useful as dielectric layers and can be used as barrier layers for copper oxide superconductors.

## EXAMPLE 7

Following the same procedure as Example 6 with 2.66 gm. strontium 1-methoxy-2-propoxide (10 mmol) and 3.40 gm. titanium isobutoxide (10 mmol) in 23 ml 1-methoxy-2-propanol, thicker films were prepared:

1 coat 609 Angstroms

2 coats 1113 Angstroms

3 coats 1777 Angstroms

4 coats 2248 Angstroms

## EXAMPLE 8

Following the same procedure as Example 6 with 3.99 gm. strontium 1-methoxy-2-propoxide (15 mmol) and 5.11 gm. titanium isobutoxide (15 mmol) in 18.5 ml 1-methoxy-2-propanol, still thicker films were prepared:

1 coat 1167 Angstroms

2 coats 2190 Angstroms

3 coats 3368 Angstroms

4 coats 4568 Angstroms

## EXAMPLE 9

A solution of strontium 1-methoxy-2-propoxide (1.33 gm.; 5 mmol) and titanium isobutoxide (1.70 gm; 5 mmol) in anhydrous 1-methoxy-2-propanol (26 ml) was prepared in a septum-sealed bottle under argon. Coatings were prepared on 7.6 cm Si wafers under these conditions:

1.5 ml solution

5000 rpm

20 secs

420° C/ 15 min

R.T./ 15 min

A total of 7 coats were applied. The wafer was cleaved in half, and half of the film was cured at 750° C for 1 hour with a 1 hour warm-up and a 6 hour cool down. The films were analyzed on a powder diffractometer. The lower temperature cured film gave no diffraction, while the higher temperature cured film had peaks which corresponded exclusively to those of SrTiO3.

EXAMPLE 10

A solution was prepared containing yttrium isopropoxide (0.45 gm.; 1.7 mmol), barium 1-methoxy-2-propoxide (1.04 gm.; 3.3 mmol) and copper 2-aminoethoxide (0.92 gm.; 5.0 mmol) in 1-methoxy-2-propanol (22 ml). A solution of water in 1-methoxy-2-propanol (10 wt %; 4.2 ml) was added. This solution was used to coat 7.6 cm Si wafers which had previously been coated with $SrTiO_3$ (1500 Angstroms) as described above. The Y-Ba-Cu coatings were spincast in a $N_2$ filled drybox. The wafers were coated with 6 layers of a yttrium-barium-copper-oxide composition (Y-Ba-Cu-O) with each layer being precured at 250°C for 10 min on a hot plate. The multicoated wafers were then cured in a quartz tube in a furnace under $N_2/H_2O$ at 825°C for 10 min with a 4 hour warm-up and a 10 hour cool down under $O_2$. The total film thickness was 4400 Angstroms for about 500 Angstroms per layer of Y-Ba-Cu-O.

EXAMPLE 11

A solution containing yttrium diethylaminoethoxide, barium diethylaminoethoxide, and copper diethylaminoethoxide was prepared with an Y:Ba:Cu molar ration of 1:2:3. The solution contained 6 wt % total metal oxides in trimethylbenzene. When spun at 2000 rpm for 60 seconds this solution yielded a poor film. Upon dilution with methoxypropanol to 3 wt % metal oxides, good films were obtained when spun at speeds from 1000-2000 rpm for 60 seconds. Single application films which were spun at 1500 rpm for 60 seconds followed by heat treatment at 350°C yielded a thickness of 500 Angstroms.

EXAMPLE 12

A solution containing yttrium methoxypropylate, barium methoxypropylate, and copper diethylaminoethoxide was prepared with an Y:Ba:Cu molar ratio of 1:2:3. The solution contained 6 wt % total metal oxides in a toluene/methoxypropanol solvent mixture. Good films were obtained when spun at speeds from 500-2500 rpm for 30 seconds. Single application films spun at 2000 rpm followed by heat treatment at 350°C in an infrared furnace yielded a thickness of 400 Angstroms. Multiple coatings are possible by curing at 400°C for 5 minutes on a hotplate between each application. Three coatings yield a thickness of 1300 Angstroms; six coatings, 2500 Angstroms.

The foregoing Examples should not be construed in a limiting sense since they have been given to illustrate only certain embodiments of the instant invention. The scope of protection is set forth in the claims which follow.

**Claims**

1. A solution which comprises:
   (a) an alkoxyalkanol solvent; and
   (b) a metal alkoxide containing ligands that are not derived from alkoxy alcohols dissolved therein.
2. A solution as claimed in Claim 1 wherein the alkoxyalkanol solvent has a total of about 3 to about 8 carbon atoms.
3. A solution as claimed in Claim 2 wherein the alkoxyalkanol has a $C_1$ to $C_4$ alkoxy substituent.
4. A solution as claimed in either Claim 2 or Claim 3 wherein the metal alkoxide contains alkyl or aryl ligands.
5. A solution as claimed in either Claim 2 or Claim 3 wherein the metal alkoxide is of the formulae $Cu(ORNH_2)_2$ or $Cu(ORNHR')_2$, where R is $C_2$-$C_4$ alkylene and R' is $C_1$-$C_4$ alkyl.
6. A solution as claimed in either Claim 2 or Claim 3 wherein the metal alkoxide is of the formula $M(ORNR'_2)x$, where M is a superconductor metal precursor, R is $C_2$-$C_3$ alkylene, R' is $C_1$-$C_8$ alkyl, and x is the valence of the metal.

7. A method of increasing the solubility of metal alkoxides which do not contain ligands derived from alkoxy alcohols which comprises dissolving the metal alkoxide in an alkoxyalkanol solvent.

8. A method as claimed in Claim 7 wherein the alkoxyalkanol solvent has a total of about 3 to about 8 carbon atoms.

9. A method as claimed in Claim 8 wherein the alkoxyalkanol has a $C_1$ to $C_4$ alkoxy substituent.

10. A method as claimed in either Claim 8 or Claim 9 wherein the metal alkoxide contains alkyl or aryl ligands.

11. A method as claimed in either Claim 8 or Claim 9 wherein the metal alkoxide is of the formulae $Cu(ORNH_2)_2$ or $Cu(ORNHR')_2$, where R is $C_2$-$C_4$ alkylene an d $R'$ is $C_1$-$C_4$ alkyl.

12. A method as claimed in either Claim 8 or Claim 9 wherein the metal alkoxide is of the formula $M(ORNR'_2)_x$, where M is a superconductor metal precursor, R is $C_2$-$C_3$ alkylene, $R'$ is $C_1$-$C_8$ alkyl, and x is the valence of the metal.

13. A method of forming metal oxide films on a substrate which comprises coating the substrate with an alkoxyalkanol solvent containing a metal alkoxide solute which does not contain ligands derived from alkoxy alcohols and heating the coating to form the metal oxide film.

14. A method as claimed in Claim 13 wherein the alkoxyalkanol solvent has a total of about 3 to about 8 carbon atoms.

15. A method as claimed in Claim 14 wherein the alkoxyalkanol has a $C_1$ to $C_4$ alkoxy substituent.

16. A method as claimed in either Claim 14 or Claim 15 wherein the metal alkoxide contains alkyl or aryl ligands.

17. A method as claimed in either Claim 14 or Claim 15 wherein the metal alkoxide is of the formulae $Cu(ORNH_2)_2$ or $Cu(ORNHR')_2$, where R is $C_2$-$C_4$ alkylene and $R'$ is $C_1$-$C_4$ alkyl.

18. A method as claimed in either Claim 14 or Claim 15 wherein the metal alkoxide is of the formula $M(ORNR'_2)_x$, where M is a superconductor metal precursor, R is $C_2$-$C_3$ alkylene, $R'$ is $C_1$-$C_8$ alkyl, and x is the valence of the metal.